# FASCICULE DE BREVET EUROPEEN

(11) **EP 1 444 324 B1**
(45) Date de publication et mention de la délivrance du brevet: **08.02.2006**
(21) Numéro de dépôt: 02776621.1
(22) Date de dépôt: 13.11.2002
(51) Int. Cl.: C12M 1/107

(54) **PROCEDE DE DESTRUCTION DE VALEURS MOBILIERES**
VERFAHREN ZUR ZERSTÖRUNG VON WERTPAPIEREN
METHOD FOR DESTROYING SECURITIES

(30) Priorité: 19.11.2001 EP 01870248
(43) Date de publication de la demande: 11.08.2004
(73) Titulaire: Sorghal asbl, 4500 Huy (BE)
(72) Inventeur: CHAPELLE, Jean, B-4560 Pailhe (BE); BIGARE, Pascal, B-5380 Fernelmont (BE); MARCHE, Christian, B-4550 Villers-le-Temple (BE); WAUTHELET, Marc, B-5640 Biesme (BE); ROMEDENNE, Raphäel, B-5502 Thynes (BE); HUBIN, Isabelle, B-4100 Seraing (BE); LOIX, Sophie, B-6230 Orbaix (BE)
(74) Mandataire: Van Malderen, Joelle
(86) Numéro de dépôt international: PCT/BE2002/000168
(87) Numéro de publication internationale: WO 2003/044157

(56) Documents cités:
- EP-A- 0 566 056
- DE-A- 19 928 663
- DE-C- 4 446 661

## Description

### Objet de l'invention

La présente invention se rapporte à un procédé de destruction irréversible de valeurs mobilières et en particulier de billets de banque par voie biologique anaérobie.

### Introduction

Les banques nationales et de façon plus générale les organismes ou les entreprises chargés de la destruction irréversible de valeurs mobilières et en particulier de billets de banque sont confrontés actuellement à des fortes contraintes telles que l'élimination des risques de reconnaissance et la dispersion des différents composants rendant impossible la détermination de leur origine.

Par valeurs mobilières, il faut entendre sans être limitatif, les billets de banque, chèques, obligations, bons de caisse, certificats immobiliers, actions et produits dérivés ou tout autre document de valeur au sens général.

Ces valeurs mobilières contiennent aujourd'hui de nombreuses caractéristiques pour lutter contre leur falsification. A titre d'exemple, nous citerons la composition particulière du support papier à base de coton. Ce papier est souvent fabriqué en étant soumis à des pressions extrêmement élevées afin d'y incruster certains éléments. Le coton, très résistant à l'usure, aux lessives et aux bactéries anaérobies et aérobies, au même titre que les vêtements en coton, est le support utilisé dans les billets de banque par exemple. On verra dans la suite que la présence de coton et d'éléments toxiques pour les bactéries tels que les métaux lourds, rendent particulièrement difficile la biodégradation de ces déchets.

Les encres d'impression utilisées pour l'impression des valeurs mobilières ont également des caractéristiques tout à fait particulières (encres à couleur changeante, encres incolores, métaux lourds entraînant une certaine toxicité, etc.) et les nombreux métaux présents sous forme de fil de sécurité, de chiffres, d'hologrammes ou de surfaces métallisées rendent la falsification par photocopie pratiquement impossible. Il y a également toute une série d'éléments chimiques inhabituels qui méritent d'être récupérés lors du processus de destruction.

Les mesures de sécurité entourant non seulement la fabrication de ces documents, mais également celle entourant leur destruction sont évidemment draconiennes.

C'est le contexte de ces contraintes qui est à l'origine de la présente invention.

Pour satisfaire aux exigences de sécurité, les responsables de l'élimination de ces valeurs mobilières procèdent généralement en plusieurs étapes.

Dans un premier temps, les documents sont découpés en fines lamelles et ensuite compactés afin d'en réduire le volume pour le transport. Cette étape se fait généralement de manière interne à ces organismes, et ceci sous haute surveillance, pour des raisons évidentes.

Dans un second temps, ces lamelles compactées sont acheminées vers leur lieu de destruction ou de stockage définitif.

Différentes méthodes de destruction de valeurs mobilières existent. Parmi les méthodes utilisées actuellement on peut citer l'incinération et la mise en décharge.

L'incinération présente l'avantage d'être peu coûteuse mais entraîne évidemment l'inconvénient que tous les éléments de valeur sont perdus. Par ailleurs, il y a également le problème des cendres contenant certains composants nécessitant souvent une mise en décharge particulière. Par ailleurs, la composition des encres est souvent très particulière et, pour des raisons évidentes de sécurité, non divulguée. Ceci entraîne une incertitude supplémentaire lors de l'incinération car ces composants peuvent donner lieu à des fumées pouvant atteindre un certain degré de toxicité.

La présente invention se fixe pour objectif de fournir une alternative à l'incinération ou à la mise en décharge de valeurs mobilières.

### Etat de la technique

Des procédés existants pour la dégradation anaérobie de déchets organiques ordinaires en plusieurs étapes sont connus et décrits notamment dans la demande de brevet EP 0566056Al de la société « REA Gesellschaft für Recycling von Energie und Abfall ». Le procédé décrit dans ce document nécessite trois étapes bien distinctes:
- la première étant l'acidification à pH < 7 ;
- la seconde étant l'hydrolyse des matières solides à pH légèrement acide ;
- la troisième étant la méthanisation des substances dissoutes.

Les substances organiques dissoutes du premier réacteur et du second réacteur alimentent un troisième réacteur. Ce procédé tente d'optimiser l'étape dite d'hydrolyse (étape déterminant la vitesse de la biométhanisàtion) afin d'augmenter le rendement de la dégradation des substances organiques solides. Le procédé peut être utilisé pour la biométhanisation de nombreuses substances organiques. Le pH étant ajusté par le contrôle des flux entre le premier et le deuxième réacteur et par la recirculation d'effluents entre le troisième et le deuxième réacteur. Les effluents du premier et du deuxième réacteur sont soumis à des séparations de phases (liquide et solide). Les deuxième et troisième réacteurs produisent du biogaz. Ce dispositif est cependant relativement complexe et incapable de digérer des déchets contenant des éléments toxiques et de fortes teneurs en coton extrêmement résistantes à la digestion biologique.

Le document DE-A-19623163 propose un dispositif de digestion de déchets d'animaux broyés qui sont hydrolysés dans un premier réacteur. La méthanogénèse se déroule dans un deuxième réacteur. Ce procédé est cependant confronté à la biodégradation de graisses et de protéines ce qui limite la recirculation à la fraction liquide du second réacteur dans le premier réacteur. Par ailleurs, le dispositif ne permet pas d'ajouter des substances neutralisantes dans le second réacteur, ce qui est indispensable pour le traitement de substrats cellulosiques tels que des billets de banques car ils acidifient la liqueur du second réacteur ce qui a pour conséquence de ralentir ou d'arrêter l'a décomposition.

La présente invention propose un système plus simple en deux étapes sans étape distincte pour l'hydrolyse des substances organiques solides. De plus, la méthanisation des valeurs mobilières telles que les billets de banque contenant des concentrations élevées en métaux lourds et en substances toxiques nécessitent des pH maintenus artificiellement élevés (par l'ajout de bases fortes). Ces pH compris entre 7,2 et 8 ne peuvent pas être obtenus uniquement par recirculation des effluents du réacteur ou par le contrôle des flux entre les réacteurs comme c'est le cas dans la demande de brevet susmentionnée.

Le brevet allemand DE 4446661C1 de B.A.N.S. Biologische Abfall- Nutzungssysteme GmbH, décrit un système adapté aux déchets alimentaires dilués ou non (jusqu'à des concentrations de 20% en matières sèches) et comportant deux étapes. Ce système ne nécessite pas l'ajout d'autres substrats (co-digestion) et est conçu pour être transportable. Les substrats sont tout d'abord réduits et homogénéisés et placés ensuite dans une cuve de stockage. La première étape comporte une pré-acidification et une hydrolyse ; la seconde étant la méthanisation. Un tiers des effluents du second réacteur est recirculé dans le premier. Le système développé ne nécessite pas de séparation liquide/solide comme cela est nécessaire dans les autres systèmes. Cette séparation n'est d'ailleurs pas possible pour les déchets alimentaires. Les effluents du système sont cependant placés dans un séparateur pour récolter séparément les solides et les eaux. Le biogaz est collecté uniquement sur le second réacteur.

Dans la présente invention la dégradation des valeurs mobilières est effectuée également en deux étapes, mais il n'y a pas de recirculation entre le second et le premier réacteur. De plus, il n'est pas nécessaire de disposer d'un pré-stockage des substrats, ce qui pose un problème logistique évident, ceux-ci étant directement mis après broyage dans la cuve d'acidification. Cette cuve est maintenue à pH acide (=5) par l'action des bactéries présentes dans l'inoculum ajouté (lisier de bovins fermentés en digesteur).

La demande de brevet DE19928663A1 de BEG BioEnergie GmbH est spécifique aux déchets organiques sans structure ou pauvre en structures, c'est-à-dire des déchets alimentaires ou d'agro-industries. Les déchets sont broyés (à l'aide d'un broyeur colloïde à vibration) pour obtenir des particules de moins de 3 mm (0,5mm) et homogénéisés. De l'eau est éventuellement ajoutée pour obtenir des concentrations en matières sèches comprises entre 5 et 22% (de préférence : 5%). Après cela, les déchets sont éventuellement soumis à des traitements biologiques, chimiques, enzymatiques ou thermiques. La première étape consiste en une hydrolyse acide durant moins de 24 heures avec aération, à 35-40°C (ou entre 35 et 60°C) et à pH acide (env. 4). Le gaz sortant est filtré par un filtre à compost. La deuxième étape est la méthanisation à 35-40°C durant moins de 10 jours et à pH supérieur ou égal à 7. Elle se déroule dans un réacteur à disque (4 rpm). Finalement, les déchets subissent une décantation dans une cuve et la partie solide est éventuellement réintroduite dans le réacteur à disques.

La méthanisation des valeurs mobilières selon la présente invention est cependant effectuée à 55°C car elle est beaucoup plus rapide qu'à 35°C. Par ailleurs, les valeurs mobilières sont riches en fibres, c'est-à-dire en structures. A 35°C, seule une faible fraction des fibres de coton est décomposée, ceci a été vérifié par des essais effectués en laboratoire sur du coton pur. Les valeurs mobilières sont également riches en structures capables de colmater des disques tels que ceux utilisés dans l'art antérieur. Le réacteur n'est pas muni de disques (permettant de fixer les bactéries sur un support) et est simplifié : les bactéries se déposent sur le fond du digesteur et sortent au fur et à mesure ; elles seront remises dans le digesteur grâce à la recirculation du décantat présent dans la cuve à effluents. De plus, le temps de séjour des déchets dans le digesteur et les concentrations employées ont été adaptées aux valeurs mobilières et ne peuvent en aucun cas correspondre à ceux cités dans le document de Bioenergie GmgH car ils occasionneraient une acidification du second réacteur entraînant l'arrêt de la méthanogénèse et une très faible décomposition des fibres. Les valeurs mobilières sont également riches en structures capables de colmater des disques tels que ceux utilisés dans la demande de brevet allemande.

Le brevet US-A-4781836 présente un système en deux étapes pour la biométhanisation de substrats organiques. Le système qui fonctionne en continu nécessite la mise en place de deux échangeurs d'anions entre le premier et le second réacteur. Ce procédé nécessite également l'utilisation d'un filtre à membrane. Ces éléments sont cependant tout à fait inadaptés pour des substrats fibreux (coton). L'échangeur d'ion doit par ailleurs être alimenté par une solution de bicarbonate pour régénérer les échangeurs d'ions.

### Buts de l'invention

La présente invention vise à fournir un procédé de destruction irréversible de valeurs mobilières simple permettant à la fois d'associer une récupération de composants valorisables et une valorisation énergétique, tout en minimisant les nuisances générées.

### Résumé de l'invention

La présente invention présente un procédé pour la destruction irréversible de valeurs mobilières préalablement découpées et/ou broyées, caractérisé en ce que lesdites valeurs sont prétraitées dans au moins une cuve d'hydrolyse et d'acidogenèse 1 maintenue à un pH inférieur à 6 et à une température comprise entre 30 et 60°C, de préférence entre 30 et 40°C, et ensuite transférées petit à petit vers une ou plusieurs cuves de méthanisation et d'acétogenèse 2 maintenues à une valeur de pH comprise entre 7,2 et 8 à l'aide de substances basiques ou de tampons, pour y être digérées en milieu anaérobie à une température comprise entre 50 et 60°C et transformées notamment en méthane et en dioxyde de carbone, la partie transférée étant immédiatement remplacée par du mélange non prétraité provenant de l'unité de broyage et de mélange 3 comprenant lesdites valeurs mobilières.

Toujours selon l'invention, les résidus liquides obtenus dans la cuve de méthanisation et d'acétogenèse sont récupérés par un système de décantation et/ou de filtration 4 et post-traités afin de récupérer les éléments valorisables et en ce que les résidus solides obtenus dans la cuve de méthanisation et d'acétogenèse 2 sont récupérés par l'unité de décantation et/ou filtration 4 et recirculés au moins partiellement dans la cuve de méthanisation 2.

Par ailleurs, la présente invention est mise en oeuvre dans un dispositif en deux étapes pour la destruction irréversible de valeurs mobilières préalablement découpées et/ou broyées caractérisé en ce que ledit dispositif comprend au moins une cuve d'hydrolyse et d'acidogenèse 1, au moins une cuve de méthanisation et d'acétogenèse 2, au moins une unité de mélange et de broyage 3 et au moins une unité de séparation et de décantation solide/liquide 4 ainsi qu'au moins une pompe 5 assurant la circulation des fluides dans les tuyaux 6 qui relient les différentes unités entre elles et qui permettent une utilisation en continu dudit dispositif.

Enfin, la présente invention divulgue l'utilisation d'une solution inoculante pour l'hydrolyse et l'acidogenèse, ainsi que pour l'acétogenèse et la méthanogenèse de valeurs mobilières préalablement découpées et/ou broyées caractérisé en ce que ladite solution est composée de bactéries issues d'une liqueur provenant d'une digestion méthanique de minimum 30 jours à 35°C de bouses de bovins ayant été nourris par des aliments riches en cellulose ou issues de réacteurs anaérobies traitant des déchets cellulosiques.

### Brève description des figures

La figure 1 représente le schéma de l'installation pilote traitant les valeurs mobilières préalablement découpées et/ou broyées.

La figure 2 représente l'unité de prétraitement effectuant l'hydrolyse et l'acidogenèse, cette unité est une partie intégrante de l'installation pilote.

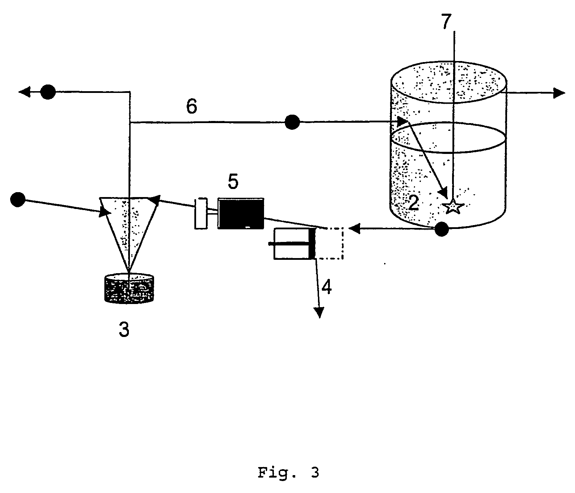

La figure 3 représente l'unité de digestion anaérobie dans laquelle s'effectue l'acétogenèse et la méthanisation, cette unité est également une partie intégrante de l'installation pilote.

### Description détaillée de l'invention

Les valeurs mobilières à détruire sont découpées en fines lamelles et compactées dans un premier stade. Le détail de cette étape de destruction ne sera pas décrit car elle est faite de manière interne à la source.

C'est donc à l'état compacté que les valeurs mobilières doivent être traitées. Dans la mesure où il s'agit de fibres cellulosiques fortement comprimées, on procède alors, de préférence, à un broyage humide de façon à obtenir des particules d'une taille suffisamment petite pour assurer la formation d'une liqueur homogène, mettre en solution le maximum dé composants et créer une surface spécifique importante pour l'attaque bactérienne.

Le produit résultant de ce broyage est alors acheminé dans une première cuve de traitement qui est ouverte ou fermée (afin de récupérer les gaz à épurer ensuite par un biofiltre), appelée cuve d'hydrolyse et d'acidogenèse 1, dans laquelle on procède en milieu anaérobie ou aérobie à une première dégradation des éléments contenus dans le produit en le mettant en contact avec une solution inoculée adéquate. Celle-ci va permettre la décomposition (l'hydrolyse) progressive, en monomères, des polymères biologiques complexes tels que la cellulose, suivi d'une transformation des monomères en acides gras volatils (acides acétique, propionique, butyrique, isovalérique, ...) et en substances métabolisables pour les bactéries, ceci constituant l'acidogenèse. Des composants minéraux sont aussi partiellement dégradés.

Lors de cette étape, les monomères formés peuvent être notamment des sucres simples et/ou des acides gras secrétés par des exoenzymes dans le milieu. Cette étape présente l'avantage de liquéfier partiellement des substances récalcitrantes par un développement naturel de certaines souches de bactéries (contenues dans l'inoculum ajouté au départ).

L'inconvénient de cette étape est cependant l'acidification de la liqueur de décomposition qui inhibe fortement les bactéries acétogènes et méthanogènes qui devront intervenir lors de la deuxième étape de digestion. L'évolution du pH (passant de plus de 7 à 5 ou 5,5) au cours de ce processus est naturelle et n'est pas influencée par l'opérateur. En cas de nécessité, de l'inoculum sera ajouté pour maintenir un pH inférieur à 6.

Après cette première étape, on passe à l'acétogenèse et à la méthanogenèse en milieu anaérobie dans une cuve fermée 2 où les monomères précédemment cités sont transformés en méthane et en gaz carbonique, notamment.

La séparation des deux étapes en deux cuves distinctes et le contrôle strict des charges (en substrats) permettent d'optimiser la dégradation et d'éviter l'inhibition des bactéries méthanigènes. Comme les essais l'ont démontré, le pH dans la cuve 2 devra être maintenu entre 7,2 et 8 par l'ajout de bases (KOH, ...)pour précipiter les métaux et pour permettre ainsi d'obtenir des conditions optimales pour la méthanogenèse. Lors des essais réalisés pour la présente invention, il a été démontré que la recirculation de la fraction liquide extraite des effluents du second réacteur ne permettait pas de maintenir un pH plus élevé que 7 dans le second réacteur. A cette façon de procéder, la recirculation de la fraction solide extraite des effluents du second réacteur a été préférée (par simple décantation ou filtration) pour obtenir une meilleure décomposition des substrats et un enrichissement en bactéries dans le second réacteur. Le pH se maintient ainsi au-dessus de 7 et des substances chimiques basiques ne sont ajoutées que lorsque le pH diminue. Il a été constaté également que la décomposition (mesuré par le rendement en biogaz) est optimale si le pH est maintenu entre 7,2 et 8. Ces pH élevés permettent également de précipiter les ions métalliques et toxiques et donc d'éviter l'empoisonnement des bactéries.

Le temps de séjour des substrats est de 15 à plus de 30 jours dans la cuve 1. Il faudra donc ajouter chaque jour un volume de substrat (broyé dans l'eau) correspondant à 3,33% et retirer une quantité équivalente. Le temps de séjour hydraulique dans la cuve 2 sera égal ou supérieur à 15 jours pour maintenir des conditions optimales pour la dégradation des substrats. Il faudra donc ajouter chaque jour un volume provenant de la cuve 1 correspondant à 6,67% et retirer une quantité équivalente. Les substrats broyés ajoutés auront une concentration de 20 à 30 g de matière sèche par litre d'eau potable.

Les conditions de température dans l'étape d'hydrolyse et d'acidogenèse se situent généralement entre 30 et 60°C, de préférence aux alentours de 35°C pour permettre le développement optimal des bactéries et éviter des surconsommations en énergie.

Après un temps de démarrage de, quelques semaines, dont la durée exacte est fortement conditionnée par le produit traité, on prélève alors journellement une quantité correspondant à environ un trentième, c'est à dire 3,3% de la première cuve de prétraitement 1 pour la transférer dans la deuxième cuve 2 étanche à l'air et remplie au départ d'un inoculum riche en bactéries méthanigènes (par exemple par l'effluent d'un digesteur méthanique agricole alimenté à 35°C par des bouses de bovins). Cette cuve est appelée cuve d'acétogenèse et de méthanogenèse 2. Cette cuve est munie d'une sortie de gaz et d'un débitmètre pour mesurer le débit de celui-ci.

Dans cette seconde étape, un mélange de bactéries va décomposer les acides gras et les métabolites en acétates et en hydrogène (acétogenèse), et produire de façon simultanée du dioxyde de carbone, et du méthane (méthanogenèse) par l'intermédiaire de souches adéquates développées dans des conditions de digestion de cellulose, soit à partir d'acétates, soit à partir de la réduction du dioxyde de carbone par l'hydrogène. Le gaz peut être récupéré pour la valorisation énergétique.

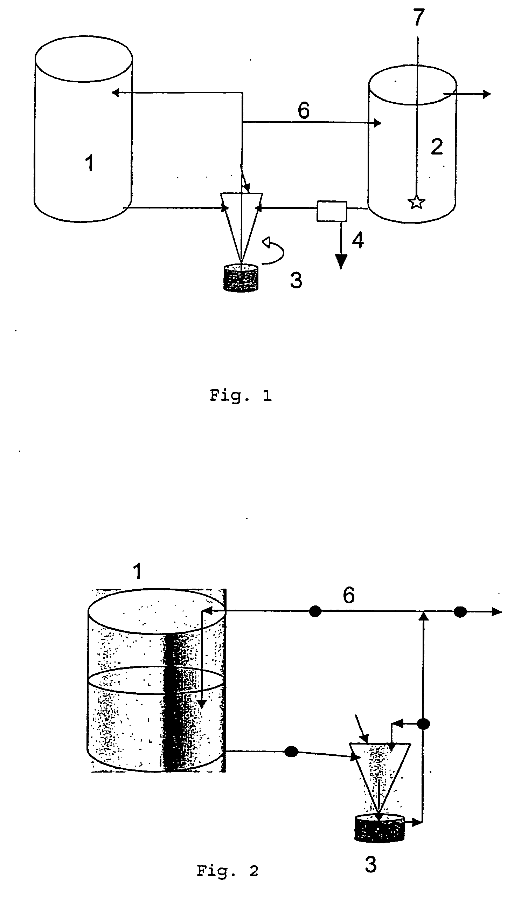

La matière soustraite à la cuve de prétraitement aérobie 1 pour alimenter la cuve 2 est immédiatement remplacée par du produit frais provenant du broyeur humide 3. Cette opération a lieu une fois par jour, ce qui permet au système de tourner en régime semi-continu comme l'illustre le schéma de la figure 1. Les deux cuves fonctionnent selon le mode infiniment mélangé : les quantités ajoutées journellement sont mélangées à l'ensemble de la cuve et les quantités ôtées (effluents) journellement proviennent de la liqueur mélangée dans la cuve. Des cloisons internes peuvent être avantageusement placées dans les cuves pour éviter de mélanger directement les produits frais avec le mélange.

Les cuves, interconnectées par des tuyaux 6 sont munies de sondes de température, de pH, d'un mélangeur et de serpentins chauffants associés à un système de régulation de température. Le mélangeur 7 est mis en route de façon périodique afin d'assurer une homogénéité parfaite de la liqueur traitée.

Une pompe 5 permet d'assurer la circulation des liqueurs dans le dispositif.

Dès le moment où la cuve d'acétogenèse et de méthanogenèse est remplie d'inoculum, on prélève au maximum 6,67% de solution par jour que l'on filtre à travers un filtre adéquat ou que l'on décante dans une cuve de stockage 4. La fraction solide des digestats (effluents) est réutilisée dans la cuve 2.

De façon générale, les bactéries méthanogènes sont très sensibles aux conditions environnementales telles que la température, le pH, la concentration en oxygène, en composants organiques et inorganiques. Ils sont sensibles aux antibiotiques et aux métaux lourds. C'est pour cette dernière raison que le pH sera maintenu par l'ajout de substances basiques (telles que le KOH (Hydroxyde de Potassium) à des valeurs élevées comprises entre 7,2 et 8.

### Description d'une forme d'exécution préférée de l'invention

Les valeurs mobilières contiennent de grandes quantités de cellulose. Le coton symbolisé par la formule (C₆H₁₀O₅)ₙ est composé à plus de 90% de cellulose.

L'inoculum utilisé est une liqueur qui a fermenté durant au minimum 30 jours à 35°C (ou plus de 15 jours à 55°C) provenant d'un digesteur méthanique alimenté par des bouses de bovins eux-mêmes essentiellement alimentés par des substrats cellulosiques (foin, paille, ...)

Les bovins alimentés de la sorte développent des souches bactériologiques capables de digérer efficacement la cellulose.

Plusieurs températures ont également été essayées à la fois pour le prétraitement et le traitement final des liqueurs. Finalement et ce, pour des raisons technico-économiques, une température approximative de 35°C pour l'étape de prétraitement, c'est à dire l'hydrolyse et l'acidogenèse et une température approximative de 55°C pour l'étape d'acétogenèse et de méthanogenèse en cuve fermée ont été retenues.

Il s'est également avéré au cours du temps que l'étape de broyage apportait beaucoup en terme d'efficacité de la fermentation et donc de gain de temps. Cette étape a donc toujours été maintenue. Le broyage fin à sec des substrats est avantageux, mais requiert plus d'énergie. Le broyage humide permet de libérer les composants des substrats et de les mettre en solution (et donc de les décomposer ensuite), ce qui peut inhiber le démarrage de la fermentation.

Pour les processus de méthanogenèse et d'acétogenèse, le pH doit être compris entre 7,2 et 8 et le rapport optimal carbone/ azote (C/N) doit être compris entre 16 et 20. Des rapports C/N beaucoup plus élevés peuvent être tolérés par les bactéries, mais en cas de carences fortes, de l'urée sera ajoutée à la liqueur de la cuve 2 (pour obtenir maximum 5% d'Azote par rapport au carbone contenu dans la liqueur). Cet ajout d'azote n'a jamais été nécessaire même après plusieurs mois d'essais en installations pilotes.

Un jeu de vannes permet de réalimenter en permanence la cuve n°2 par de la liqueur en provenance de la cuve n°1 par l'équivalent de la quantité qui a été soustraite lors de chaque cycle et qui sera d'ailleurs remplacé à la base par la quantité équivalente provenant du broyeur humide.

Au point de départ, dans la cuve de prétraitement n°1 qui a une capacité utile de 300 U (Unités de volume égale à un litre, un décalitre ou un m³), on introduit un mélange constitué de 30 U d'inoculum provenant de digesteurs (de la ferme expérimentale du Centre Technique Agricole de Strée en Belgique, par exemple, ou d'un autre réacteur anaérobie) et de 270 U d'eau ; le mélange est chauffé à environ 35°C durant plus d'une semaine et on ajoute ensuite 25 g de substrat broyé (valeurs mobilières) par litre de mélange eau-inoculum.

Le mélange eau-inoculum-substrat est toujours maintenu à 35°C dans la cuve 1. Après 30 jours (démarrage), 3,3% du volume (soit 10 U) sont extraits journellement pour être ajouté à la cuve 2 et une quantité équivalente (10 U) de mélange substrat broyé (25g/l) frais - eau sont ajoutés journellement. Dans la cuve 2 d'une capacité utile de plus de 150 U, au point de départ, il y a 150 U d'inoculum.

**Légende**
1. cuve d'hydrolyse et d'acidogenèse
2. cuve de méthanisation et d'acétogenèse
3. unité de mélange et de broyage accueillant les valeurs mobilières et l'eau ou l'effluent
4. unité de stockage et de séparation solide/liquide
5. pompe de circulation
6. tuyaux de liaison notamment pour la recirculation des décantats
7. mélangeur

## Revendications

1. Procédé pour la destruction irréversible de valeurs mobilières préalablement découpées et/ou broyées, **caractérisé en ce que** lesdites valeurs sont prétraitées dans au moins une cuve d'hydrolyse et d'acidogenèse (1) maintenue à un pH inférieur à 6 et à une température comprise entre 30 et 60°C, de préférence entre 30 et 40°C, et ensuite transférés petit à petit vers une ou plusieurs cuves de méthanisation et d'acétogenèse (2) maintenues à une valeur de pH comprise entre 7,2 et 8 à l'aide de substances basiques ou de tampons, pour y être digérées en milieu anaérobie à une température comprise entre 50 et 60°C et transformés notamment en méthane, et en dioxyde de carbone, la partie transférée étant immédiatement remplacée par du mélange non prétraité provenant de l'unité de broyage et de mélange (3) comprenant lesdites valeurs mobilières.

2. Procédé selon la revendication 1, **caractérisé en ce que** les résidus liquides obtenus dans la cuve de méthanisation et d'acétogenèse sont récupérés par un système de décantation et/ou de filtration (4) et post-traités afin de récupérer les éléments valorisables et **en ce que** les résidus solides obtenus dans la cuve de méthanisation et d'acétogenèse (2) sont récupérés par l'unité de décantation et/ou filtration (4) et recirculés au moins partiellement dans la cuve de méthanisation (2).

3. Utilisation d'une solution inoculante pour l'hydrolyse et l'acidogenèse, ainsi que pour l'acétogenèse et la méthanogenèse de valeurs mobilières préalablement découpées et/ou broyées **caractérisé en ce que** ladite solution est composée de bactéries issues d'une liqueur provenant d'une digestion méthanique de minimum 30 jours à 35°C de bouses de bovins ayant été nourris par des aliments riches en cellulose ou issues de réacteurs anaérobies traitant des déchets cellulosiques.

## Claims

1. Method for the non-reversible destruction of securities previously cut and/or crushed, **characterised in that** said securities are pre-treated in at least one hydrolysis and acidogenesis tank (1) maintained at a pH lower than 6 and at a temperature between 30 and 60°C, preferably between 30 and 40°C, and then gradually transferred into one or several methanation and acetogenesis tanks (2) maintained at a pH value between 7.2 and 8 by means of alkaline substances or buffers, in order to be digested there in an anaerobic environment at a temperature between 50 and 60°C and transformed in particular into methane and carbon dioxide, the transferred part being immediately replaced by non pre-treated mixture originating from the crusher and mixture unit (3) containing said securities.

2. Method according to Claim 1, **characterised in that** the liquid residues obtained in the methanation and acetogenesis tank are recovered by a decantation and/or filtration system (4) and post-treated in order to recover recoverable elements and **in that** the solid residues obtained in the methanation and acetogenesis tank (2) are recovered by the decantation and/or filtration unit (4) and at least partially recirculated in the methanation tank (2).

3. Use of an inoculating solution in order to produce hydrolysis and acidogenesis as well as the acetogenesis and methanation of securities previously cut and/or crushed, **characterised in that** said solution comprises bacteria sourced from a liquor originating from the methane digestion of minimum 30 days at 35°C of cattle manure, which been fed cellulose-rich food or issued from anaerobic reactors treating cellulose waste.

## Patentansprüche

1. Verfahren für die endgültige Vernichtung von vorher zerschnittenen und/oder zerkleinerten Wertpapieren, **dadurch gekennzeichnet, dass** die besagten Wertpapiere in mindestens einer Hydrolyse- und Acidogenesewanne (1) mit einem pH-Wert, der weniger als 6 beträgt, und einer Temperatur zwischen 30 und 60 °C, vorzugsweise zwischen 30 und 40 °C, vorbehandelt werden und anschließend nach und nach mit Hilfe von basischen Substanzen oder Puffern in eine oder mehrere Methanogenese- und Acetogenesewannen (2) mit einem pH-Wert zwischen 7,2 und 8 weitergeleitet werden, um dort in einem anaeroben Milieu bei einer Temperatur zwischen 50 und 60 °C aufgeschlossen und insbesondere in Methan und in Kohlendioxid umgewandelt zu werden; dabei wird der weitergeleitete Teil unverzüglich durch eine nicht vorbehandelte Mischung ersetzt, die aus der Zerkleinerungs- und Mischeinheit (3) stammt, die die besagten Wertpapiere enthält.

2. Verfahren gemäß Anspruch 1, **dadurch gekennzeichnet, dass** die flüssigen Reststoffe in der Methanogenese- und Acetogenesewanne von einem Dekantierungs- und/oder Filtersystem (4) aufgefangen werden und nachbehandelt werden, so dass die verwertbaren Bestandteile wiederverwertet werden können, und **dadurch gekennzeichnet, dass** die festen Reststoffe in der Methanogenese- und Acetogenesewanne (2) von dem Dekantierungs- und/oder Filtersystem (4) aufgefangen werden und zumindest teilweise wieder in die Methanogenesewanne (2) eingeleitet werden.

3. Verwendung einer Schutzlösung für die Hydrolyse und die Acidogenese sowie für die Acetogenese und die Methanogenese von vorher zerschnittenen und/oder zerkleinerten Wertpapieren, **dadurch gekennzeichnet, dass** sich die besagte Lösung aus Bakterien zusammensetzt, die aus einer Flüssigkeit hervorgehen, die bei einer Methangärung von mindestens 30 Tagen bei 35 °C von Rindermist, die mit zellulosereichem Futter versorgt wurden, entstanden ist, oder aus anaeroben Reaktoren, in denen zellulosehaltige Abfälle behandelt werden.
